# EUROPÄISCHE PATENTSCHRIFT

(11) **EP 1 315 553 B1**
(45) Veröffentlichungstag und Bekanntmachung des Hinweises auf die Patenterteilung: **06.12.2006**
(21) Anmeldenummer: 01978134.3
(22) Anmeldetag: 05.09.2001
(51) Int. Cl.: B01D 61/18, B01D 63/08, G01N 33/49

(54) **VORRICHTUNG UND VERFAHREN ZUR SEPARATION VON UNGELÖSTEN BESTANDTEILEN AUS BIOLOGISCHEN FLÜSSIGKEITEN**
DEVICE AND METHOD FOR SEPARATING UNDISSOLVED CONSTITUENTS OUT OF BIOLOGICAL FLUIDS
DISPOSITIF ET PROCEDES POUR SEPARER DES CONSTITUANTS NON DISSOUS CONTENUS DANS DES LIQUIDES BIOLOGIQUES

(30) Priorität: 08.09.2000 DE 10046173
(43) Veröffentlichungstag der Anmeldung: 04.06.2003
(73) Patentinhaber: peS Gesellschaft für medizinische Diagnose-Systeme mbH, 04416 Markkleeberg (DE)
(72) Erfinder: RAUCH, Peter, 48301 Nottuln (DE); KATERKAMP, Andreas, 48149 Münster (DE); SCHMITZ, Marco, 48565 Steinfurt (DE); GRAWE, Frank, 48607 Ochtrup (DE); MEUSEL, Markus, 48159 Münster (DE)
(74) Vertreter: Pfenning, Meinig & Partner GbR
(86) Internationale Anmeldenummer: PCT/DE2001/003517
(87) Internationale Veröffentlichungsnummer: WO 2002/020141

(56) Entgegenhaltungen:
- EP-A- 0 785 012
- US-A- 4 212 742
- US-A- 4 639 316
- US-A- 4 970 052
- US-A- 5 135 716
- US-A- 5 135 719

## Beschreibung

Die Erfindung betrifft eine Vorrichtung und Verfahren zur Separation von ungelösten Bestandteilen aus biologischen Flüssigkeiten, insbesondere für die Separation von Blutplasma aus Vollblut. Es kann aber auch die Separation von zellulären Bestandteilen aus Zellkulturüberständen durchgeführt werden, um lediglich gelöste Bestandteile enthaltende Zellflüssigkeit zu erhalten. Weitere Beispiele für biologische Flüssigkeiten sind Blutserum, Urin und Liquor oder andere Körperflüssigkeiten, so dass mit der Erfindung reine von ungelösten Bestandteilen freie Flüssigkeiten, z.B. zu Analsysezwecken zur Verfügung gestellt werden kann.

Die Erfindung ist besonders für die labormedizinische Diagnostik geeignet. Dabei werden zu Analysezwecken relativ geringe Mengen an biologischer Flüssigkeit, z.B. Blutplasma benötigt, die weitestgehend frei von störenden Komponenten sind. Solche störenden Komponenten sind insbesondere zelluläre Bestandteile, beispielsweise Leukozyten und Erythrozyten.

Entsprechend reines Blutplasma kann bei verschiedenen bekannten Diagnoseverfahren, wie z.B. den sogenannten Immuno-Assays eingesetzt werden.

Üblicherweise wird die Separation von Blutplasma aus Vollblut durch zentrifugieren durchgeführt, was besonders aufwendig und kostenintensiv ist.

Bei immunchromatografischen Schnelltests werden standardmäßig Separationsmembranen verwendet, wenn z.B. Vollblut als Probenflüssigkeit benutzt wird. Das separierte Blutplasma bleibt dabei aber generell innerhalb des Membranmaterials und liegt demzufolge nicht als reine Flüssigkeit ohne Träger vor, was die quantitative Analyse in den meißten Fällen unmöglich macht.

Desweiteren ist es aus EP 0 336 483 B1 bekannt, einen zweiteiligen Aufbau aus hydrophiler Mikroporen-Trennmembran und hydrophiler Mikroporen-Sammelmembran für solche Zwecke einzusetzen. Dabei werden mit einem solchen Zweimembranensystem erst mit der Trennmembran Hämatokrit und Blutplasma getrennt und das separierte Blutplasma in der Sammelmembran gesammelt. Die das Blutplasma enthaltende Sammelmembran wird anschließend von der Trennmembran getrennt und die Analyse von Blutplasmakomponenten mit der Sammelmembran durchgeführt, was Probleme beim Handling mit sich bringt und bestimmte Analyseverfahren, insbesondere quantitative Analyseverfahren, bei denen eine Messung in einem reinen Flüssigkeitsvolumen und nicht innerhalb einer Membran durchgeführt wird, nicht ohne weitere Behandlung benutzt werden können.

Aus EP 0 785 012 A1 ist es bekannt, eine Separation durch eine reine Filtration durchzuführen. Hierzu werden eine Glasfaser- und eine mikroporöse Membran benutzt, durch die das Blutplasma geführt und die störenden Zellkomponenten ausgefiltert werden. Bei einer solchen Filtration setzen sich aber die Mikroporen der Membran sehr schnell, insbesondere durch Erythrozyten zu. Die für die Filtration erforderliche Zeit ist relativ lang, da wenn überhaupt nur mit kleinen Druckgradienten zwischen beiden Seiten der Filtermembran gearbeitet werden kann, um eine Hämolyse der Blutzellen und entsprechend eine Verunreinigung des separierten Blutplasma zu vermeiden.

In US 5,135,719 ist eine Lösung beschrieben, bei der Blutplasma aus Vollblutproben separiert werden soll. Dabei wird das Vollblut auf ein Filter gegeben. Beim Durchströmen des Filters orthogonal zur Oberfläche erfolgt eine Separation fester Bestandteile und das Blutplasma wird dann über Kanäle infolge wirkender Kapillarkräfte abgezogen.

Bei einer in US 4,970,052 beschriebenen Vorrichtung soll Vollblut über einen Kanal entlang einer Membranoberfläche strömen. Die Membran trennt diesen Kanal für Vollblut von einem zweiten Kanal für separiertes Blutplasma, das infolge einer Druckdifferenz durch die Membran, orthogonal zur angeströmten Oberfläche der Membran in den zweiten Kanal für Blutplasma gelangt und von dort abgezogen werden kann.

Ein ähnliches Prinzip geht aus US 4,639,316 hervor.

Aus US 4,212,742 ist eine Filtervorrichtung zur Separation von Blutzellen enthaltenden flüssigen Suspensionen bekannt. Auch hier durchströmt die jeweilige Flüssigkeit Kanäle und die Separation soll mit Hilfe von zur Filtration geeigneten Membranen erfolgen, durch die die jeweilige Flüssigkeit wieder orthogonal zur jeweiligen Oberfläche der Membranen für die Separation hindurchströmen soll.

Aufgabe der Erfindung ist es, eine einfache und kostengünstige Möglichkeit vorzuschlagen, mit der ungelöste Bestandteile aus biologischen Flüssigkeiten, insbesondere Blutplasma aus Vollblut separiert werden können und die biologische Flüssigkeit danach als reines Flüssigkeitsvolumen ohne Träger vorliegt.

Erfindungsgemäß wird diese Aufgabe mit einer die Merkmale des Anspruchs 1 aufweisenden Vorrichtung und einem Verfahren gemäß Anspruch 27 gelöst. Vorteilhafte Ausgestaltungen und Weiterbildungen können mit den in den untergeordneten Ansprüchen genannten Merkmalen erreicht werden.

Nachfolgend wird ausschließlich auf Vollblut, aus dem von ungelösten Bestandteilen freies Blutplasma separiert werden soll, als ein Beispiel für eine biologische Flüssigkeit Bezug genommen, wobei mit anderen biologischen Flüssigkeiten selbstverständlich auch analog verfahren werden kann.

Bei der erfindungsgemäßen Lösung wird z.B. Vollblut ggf. unter Zugabe von gerinnungshemmenden Mitteln in einen Aufgaberaum gegeben. Der Aufgaberaum ist vollflächig und passgenau mittels einer Membran von einem an sich geschlossenen Hohlraum mit geringer Höhe getrennt. Der Hohlraum ist an einen Flußkanal bzw. eine Öffnung, aus dem/der das separierte Blutplasma entnommen werden kann, angeschlossen.

Mit der Membran erfolgt die Separation vollständig bzw. nahezu vollständig nach chromatographischem Prinzip, wobei die Bestandteile der Flüssigkeit bzw. des Vollbluts durch die Membran mit unterschiedlichen Geschwindigkeiten transportiert werden und das Blutplasma schneller, als z.B. die im Vollblut enthaltenen zellulären Bestandteile durch die Membran strömt. Die Bewegungsrichtung kann dabei orthogonal zur eigentlichen Membranebene einer Membran sein.

Da das Blutplasma schneller durch die Membran gelangt, kann es mittels des auf der anderen Membranseite ausgebildeten sich vorteilhaft verjüngenden Bereich des Hohlraums in Richtung auf einen sich anschließenden Flußkanal bzw. eine Öffnung strömen und dort entnommen oder gesammelt werden und anschließend als reines Flüssigkeitsvolumen einer Analyse zugeführt werden. Der sich verjüngende Bereich des Hohlraumes ist vorteilhaft außerhalb des von der nur separierenden Membran abgedeckten Bereiches angeordnet.

Da sich das Blutplasma an der Seite der Membran, die dem Hohlraum geringer Höhe zugewandt ist, in der Membran sammelt und durch Kapillarkräfte in ihr gehalten wird, müssen entsprechende Kräfte wirken, mit denen das Blutplasma aus der Membran heraus gelangt. Dies können Saug-, Druck-, Kapillarkräfte oder der durch die aufgegebene Vollblutprobe wirkende hydrostatische Druck sein, wobei auch eine Kombination mehrerer dieser Kräfte und Drücke anwendbar ist. Ein hydrostatischer Druck wirkt aufgrund der Flüssigkeitssäule oberhalb der Separationsmembran.

Dabei spielt die Form und Dimensionierung des Hohlraumes, insbesondere seine geringe über der gesamten Fläche gleiche Höhe, die kleiner als 1 mm ist, bevorzugt im Bereich von 0,01 bis 0,5 mm und besonders bevorzugt bei 0,05 mm liegen sollte, eine vorteilhafte Rolle.

Die Separation, der Transport und/oder das Abziehen des Blutplasma aus der Vorrichtung kann auch mit Unterstützung von Saug- oder Druckkräften erfolgen, wobei dies auch bei der nachfolgend beschriebenen, alternativen Ausgestaltung der Erfindung der Fall sein kann.

Erfindungsgemäß ist eine Membran vorhanden, mit der ein lateraler Transport des Blutplasma innerhalb dieser Transportmembran zur Öffnung bzw. dem Flußkanal erfolgt. Diese Transportmembran ist in den Hohlraum geringer Höhe eingesetzt werden und sollte diesen möglichst vollflächig ausfüllen und kann mit der Oberfläche der Unterseite der ausschließlich separierenden Membran in Kontakt stehen. Diese Transportmembran ist so ausgewählt, dass sie eine höhere Kapillarkraftwirkung, als die ausschließlich zur Separation genutzte Membran erreicht, so dass das Blutplasma aus der Separationsmembran durch einen Kapillarkraftanstieg in die Transportmembran gelangen kann und in dieser Transportmembran lateral und damit orthogonal zur Separationsrichtung der Separationsmembran transportiert wird.

Bei Auswahl eines geeigneten Membranmaterials wird die Transportmembran auch genutzt, um zusätzlich unerwünschte Komponenten u.U. auch selektiv zu separieren.

Die erfindungsgemäße Vorrichtung ist so ausgebildet, dass zwischen einem Aufgaberaum für die Flüssigkeit, aus der die ungelösten Bestandteile separiert werden sollen und einem Flußkanal oder einer Öffnung, durch die die entsprechend separierte Flüssigkeit in ein Volumen überführt werden kann, lediglich die Transportmembran zumindest im Hohlraum geringer Höhe angeordnet ist, die sowohl Transportfunktion für die jeweilige Flüssigkeit erfüllt, wie auch die ungelösten Bestandteile aus der Flüssigkeit separiert. Dabei wird mit einer solchen Transportmembran, zumindest aufgrund ihr eigener Kapillarkraftwirkung die Flüssigkeit ausgehend vom Aufgaberaum durch die Transportmembran in Richtung auf den Flußkanal bzw. eine Öffnung transportiert. Die ungelösten Bestandteile werden mittels dieser Transportmembran chromatografisch separiert, so dass aus dem Flußkanal oder der Öffnung von ungelösten Bestandteilen freie Flüssigkeit abgenommen werden kann. Dabei wird die für die Separation erforderliche Zeit und das Flüssigkeitsvolumen von den Eigenschaften des Materials der Transportmembran, deren lateraler Länge, der Dicke der Transportmembran bzw. der Höhe des Hohlraumes geringer Höhe bestimmt. Diese Parameter können zusätzlich durch angelegte Druck- und/oder Saugkräfte beeinflußt werden.

Eine so ausgebildete erfindungsgemäße Vorrichtung ist insbesondere für die Gewinnung relativ kleiner von ungelösten Bestandteilen befreiter Flüssigkeitsvolumina, im Bereich einiger weniger µl, geeignet.

Die Zeit und das erreichbare Flüssigkeitsvolumen pro Zeiteinheit können auch dadurch beeinflußt werden, dass am Ende der Transportmembran, das in Richtung auf den Flußkanal bzw. die Öffnung weist, parallel zur Strömungsrichtung der Flüssigkeit, also in lateraler Richtung Einschnitte ausgebildet sein können, die jedoch in ihrer Länge begrenzt sind und nicht über die Gesamtlänge der Transportmembran führen.

Bei der bis hier beschriebenen Ausführungsform einer erfindungsgemäßen Vorrichtung, bei der lediglich eine solche Transportmembran verwendet werden soll, gelangt die zu separierende Flüssigkeit aus dem Aufgaberaum über die eine in Richtung auf den Aufgaberaum zeigende Stirnfläche der Transportmembran für den lateralen Transport und die Separation in die Transportmembran.

Es besteht aber auch die Möglichkeit, die Transportmembran so zu konturieren und zu dimensionieren, dass sie sowohl den Hohlraum geringer Höhe, wie auch die gesamte Fläche des Aufgaberaumes flächig ausfüllt. In diesem Fall gelangt die zu separierende Flüssigkeit über die freie Oberfläche der Transportmembran, im Bereich des Aufgaberaumes in die Transportmembran und wird von dort in lateraler Richtung zum Flußkanal bzw. der Öffnung in der Transportmembran durch den Hohlraum geringer Höhe gefördert. Dabei ist die Geschwindigkeit der ungelösten Bestandteile in der Transportmembran kleiner, so dass über einen gewissen Zeitraum in den Flußkanal bzw. an der Öffnung reine Flüssigkeit eintreten bzw. austreten oder in ein Volumen überführt werden kann.

Im Übrigen kann ein so ausgeführtes Beispiel einer erfindungsgemäßen Vorrichtung so ausgebildet sein, wie dies bereits vorab und auch nachfolgend beschrieben worden ist bzw. beschrieben wird.

Geeignete Membranen für die chromatografische Separation von Blutplasma sind mehrschichtige z.B. dreischichtige Polyestermembranen, wie sie von der Firma Pall unter der Handelsbezeichnung "Hemasep V" erhältlich sind.

Für die im Hohlraum optional angeordnete Transportmembran können solche, die den Transport von Blutplasma mittels Kapillarkräften bewirken eingesetzt werden. Hierfür können Fasermembranen aus natürlichen und synthetischen Fasern eingesetzt werden. Als besonders geeignet hat sich dabei die ebenfalls von der Firma Pall unter dem Handelsnamen "CytoSep 1660 oder 1661" erhältliche Membran, insbesondere in Kombination mit der ausschließlich separierenden Membran "Hemasep V" herausgestellt. Mit diesem und den Membrantypen "CytoSep 1660, 1662, 1663 oder Hemasep L" kann auch beim lateralen Transport weiter separiert werden.

Es können aber auch reine Transportmembranen, wie z.B. Nylonmembranen (Nylon 6,6), Cellulosemembranen, Nitrocellulosemembranen, Polyethersulfonmembranen, Borsilicatmembranen und Glasfasermembranen eingesetzt werden, die aber eine verringerte Blutplasmaausbeute oder einen geringeren Reinheitsgrad des Blutplasma erreichen.

Das durch die erste, den Aufgaberaum und den Hohlraum trennende Membran separierte Blutplasma liegt an der Unterseite dieser Membran vor und kann von dort durch wirkende Kapillarkräfte, infolge der Form und der Höhe, ggf. mit Unterstützung der weiteren im Hohlraum angeordneten Transportmembran durch hydrostatische Kräfte in ein Volumen überführt werden.

So kann eine für Analysen in der Regel ausreichende Blutplasmamenge innerhalb eines Zeitraumes von 10 und mehr Minuten erhalten werden.

Die erforderliche Separationszeit kann aber deutlich verkürzt werden, wenn zusätzlich Saug- und/oder Druckkräfte ausgenutzt werden. In diesem Fall sollte der Zeitraum für die Separation möglichst nicht größer als
10 min sein, um zu sichern, dass reines Blutplasma im Volumen vorliegt.

Es kann aber auch durch Anlegen von Unterdruck eine Saugkraft ausgenutzt werden. Hierzu kann an der Öffnung oder dem Ausgang eines Flußkanals eine Kolben-Zylindereinheit, z.B. eine herkömmliche Spritze angesetzt werden. Durch entsprechende Bewegung des Kolbens im Zylinder liegt eine Saugkraft sowohl im Hohlraum, wie auch an der Unterseite der eigentlichen Separationsmembran an, mit der die erforderliche Zeit bis auf wenige Minuten verkürzt werden kann. Das reine, separierte Blutplasma kann unmittelbar im Zylinder aufgenommen und mit dem Zylinder zu einem Analyseort transportiert werden.

Es kann aber auch eine Druckkraft auf die jeweilige Probe, die in den Aufgaberaum gegeben worden ist, allein oder zusätzlich ausgeübt werden um das Separieren zeitlich zu verkürzen. Dabei kann ein Stempel oder Kolben auf die Flüssigkeitsoberfläche aufgesetzt und mit der Gravitationskraft oder ggf. zusätzlicher Kräfte gegen die Probenflüssigkeit und Membranoberfläche drücken. Der gleiche Effekt kann aber auch mit einem komprimierten, bevorzugt inertem Gas, das in den nach Befüllen geschlossenen Aufgaberaum gedrückt wird, erreicht werden. Dabei sollte die gesamte Membranoberfläche im Aufgaberaum mit Probenflüssigkeit (Vollblut) bedeckt sein.

Der an sich an einer Seite offene Aufgaberaum kann aber auch nach dem Befüllen mit der Probe mit einem flexiblen Material, z.B. einer Folie verschlossen werden und durch einfaches Drücken von Hand die gewünschte senkrecht auf die Oberfläche der Membran wirkende Druckkraft, infolge der erreichten Volumenverringerung aufgebracht werden.

Der Hohlraum geringer Höhe, der zwischen der eigentlichen Separationsmembran und der Öffnung bzw. dem Flußkanal angeordnet ist, stellt ein Interface zwischen diesen Elementen dar und dient zum Transport des separierten Blutplasma in ein geeignetes Volumen.

In der Regel wird an einem solchen spaltförmigen Hohlraum eine Verjüngung in Richtung auf eine Öffnung bzw. den Flußkanal ausgebildet sein. Es ist aber auch denkbar zwei sich diametral gegenüberliegende, verjüngende oder mehrere beispielsweise sternförmig angeordnete sich verjüngende Bereiche am Hohlraum auszubilden, die in Flußkanäle oder Öffnungen münden und mit dem Hohlraum geringer Höhe kommunizieren. So kann die Separationszeit verkürzt und/oder die Blutplasmamenge erhöht werden.

Der Hohlraum geringer Höhe sollte bis auf den Bereich des Aufgaberaumes und der Öffnung durch die separierte Flüssigkeit direkt in ein Volumen überführt oder im Bereich der Öffnung, die mit einem Flußkanal kommuniziert bzw. eine Öffnung in einer Transportmembran ausgebildet ist und separierte Flüssigkeit durch den Flußkanal in ein Volumen überführt wird, flüssigkeitsdicht abgeschlossen sein, um ein unerwünschtes Entweichen von Flüssigkeit zu vermeiden und den Flüssigkeitsstrom gezielt zu den Öffnungen zu richten.

In jedem Fall wirkt sich aber, die relativ große zur Verfügung stehende Fläche der Aufgaberaum und Hohlraum trennenden Separationsmembran in diesem Sinne vorteilhaft aus.

Mit der Erfindung kann die für die Separation erforderliche Zeit verkürzt werden. Eine entsprechende Vorrichtung ist einfach aufgebaut und kostengünstig herstellbar. Sie kann sehr einfach genutzt werden. Die Separation erfolgt schonend und das Blutplasma ist weitestgehend rein, liegt als flüssige Phase, ohne störendes Membranmaterial vor und ist so für die verschiedensten Analyseverfahren geeignet.

Nachfolgend soll die Erfindung an Hand eines Beispiels näher erläutert werden.

Dabei zeigen:
- Figur 1: ein Beispiel einer erfindungsgemäßen Vorrichtung in einer Einzelteildarstellung;
- Figur 2: eine Seitenansicht des Beispiels nach Figur 1 im Schnitt;
- Figur 3: eine Draufsicht auf das Beispiel einer erfindungsgemäßen Vorrichtung;
- Figur 4: eine Seitenansicht einer Vorrichtung mit zusätzlicher Transportmembran im Schnitt und
- Figur 5: ein Beispiel einer Vorrichtung mit einem Zwischenbehälter.

Das nachfolgend beschriebene Beispiel einer erfindungsgemäßen Vorrichtung ist relativ einfach aufgebaut und kann beispielsweise aus wenigen Kunststoffspritzgussteilen kostengünstig hergestellt werden.

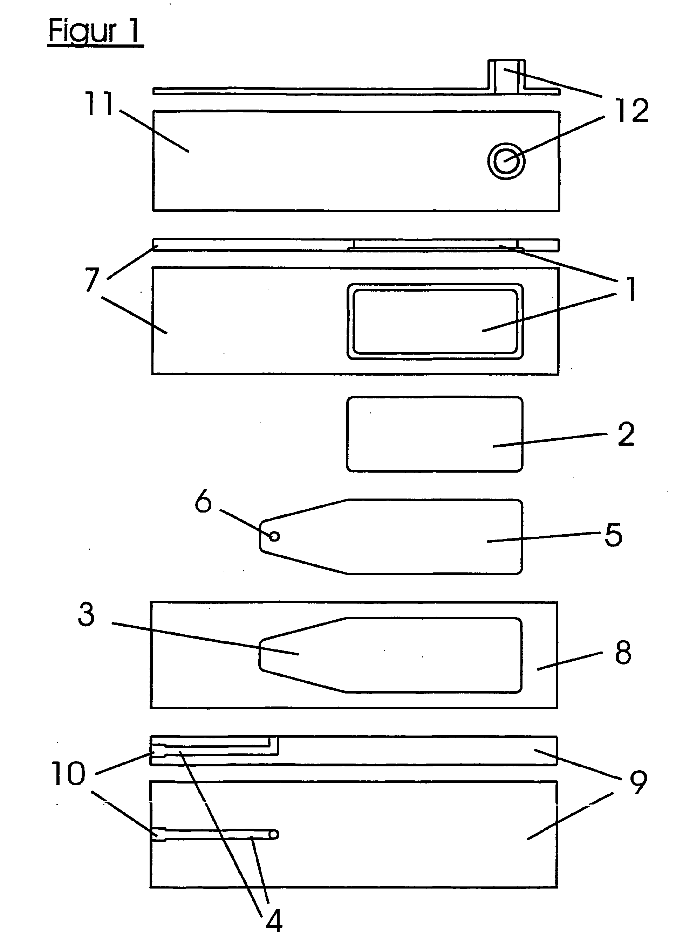

In Figur 1 sind die bei diesem Beispiel verwendeten einzelnen Elemente in einer Einzelteildarstellung gezeigt.

Hier wird ein Deckelteil 7 mit einer einen Aufgaberaum 1 bildenden Öffnung verwendet, wobei die Dicke des Deckelteils 7 und die freie Querschnittsfläche der Öffnung, das für die Probenflüssigkeit zur Verfügung stehende Volumen im Aufgaberaum 1 vorgeben.

Nach unten ist dieses Beispiel einer erfindungsgemäßen Vorrichtung mit einem Basisteil 9 ausgebildet. Deckelteil 7 und Basisteil 9 werden vor dem Gebrauch miteinander verbunden. Beide Teile können verklebt, verschweißt oder form- und kraftschlüssig durch beipielweise Clips miteinander verbunden werden.

Sie können aus Kunststoff im Spritzgußverfahren hergestellt, aber auch aus anderen Materialien bestehen.

Der sich in seiner Breite verjüngende Hohlraum 3 geringer Höhe kann durch entsprechende Ausarbeitung in einer Fläche des Deckel- oder Basisteiles 7 oder 9, die aufeinander zu gerichtet ist, ausgebildet sein.

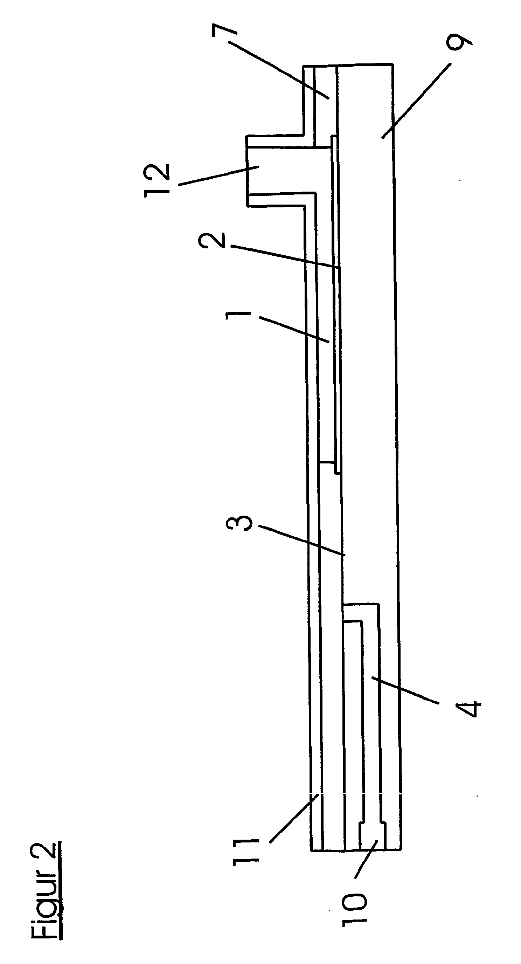

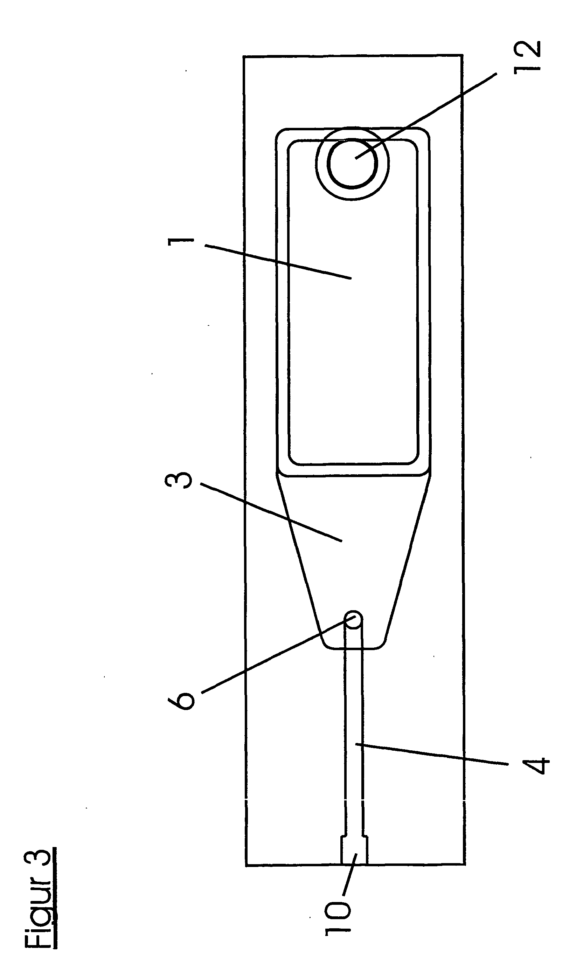

Beim in den Figuren 1 bis 3 gezeigten Beispiel wird aber ein Klebefilm 8, mit dem Deckelteil 7 und Basisteil 9 verbunden werden, verwendet und bildet durch einen ausgestanzten Teil den sich einseitig keilförmig verjüngenden Hohlraum 3 geringer Höhe aus. Der hier verwendete Klebefilm 8 hat eine Dicke von 0,13 mm und gibt die Hohlraumhöhe vor.

Der Hohlraum 3 ist flächig so dimensioniert, dass die Querschnittsfläche des Aufgaberaumes 1 vollständig überdeckt ist und sich zusätzlich ein sich verjüngender Teil anschließt, der nicht von der Membran 2 überdeckt ist.

In den Aufgaberaum 1 ist die Membran 2 zur Separation des Blutplasma so eingesetzt, dass eine flüssige Probe auf die Oberfläche der Membran 2 in den Aufgaberaum 1 gegeben werden kann, ohne dass Probenflüssigkeit unsepariert in den Hohlraum 3 gelangen kann.

Die bei diesem Beispiel verwendete Membran 2 ist eine "Hemasep V"-Membran, die eine Länge von 30 mm, eine Breite von 13 mm und eine Dicke von 0,89 ± 0,05 mm hat.

Bei diesem Beispiel wird eine Transportmembran 5 verwendet, die den Hohlraum 3 vollflächig ausfüllt. Diese Transportmembran 5 hat bei diesem Beispiel eine Länge von 45 mm und ist ebenfalls 13 mm breit. Die kleinsten Breiten von Transportmembran 5 und Hohlraum 3 im verjüngten Bereich beträgt 5 mm, bei einem Winkel der Verjüngung von ca. 15°.

Im Basisteil 9 ist ein Flußkanal 4, auf den ggf. auch verzichtet werden kann, ausgebildet, durch den das separierte Blutplasma zur Öffnung 10 geführt wird. Durch eine Öffnung, die im sich verjüngenden Bereich des Hohlraumes 3 geringer Höhe angeordnet ist, gelangt das lateral durch die Transportmembran 5 transportierte Blutplasma in den Flußkanal 4 und kann von dort abgezogen werden. In der Transportmembran 5 ist eine Öffnung 6 ausgebildet, die mit der Einlaßöffnung des Flußkanals 4 kommuniziert.

Um diese Öffnung 6 sammelt sich das separierte Blutplasma in der Transportmembran 5 und kann von dort durch wirkende Druck- oder Saugkräfte in ein geeignetes Volumen abgezogen werden. So kann eine Saugkraft über die Öffnung 10 wirken, um dies zu erreichen. Wegen der kleinen Dimensionen der Öffnung sind entsprechend kleine Kräfte erforderlich. Eine Saugkraft wirkt auf die relativ kleine Innenrandfläche der in der Transportmembran 5 ausgebildeten Öffnung 6, die maßgeblich von der Dicke der Transportmembran 5 bestimmt wird.

An die Öffnung 10 des Flußkanals 4 kann eine entsprechend gestaltete Kanüle einer Spritze angesetzt werden und das so saugkraftunterstützt separierte Blutplasma in den Zylinder gezogen werden.

Die Transportmembran 5 kann aus einem im allgemeinen Teil der Beschreibung genannten Material gebildet sein.

Zur Separation von Blutplasma kann eine Vollblutprobe ca. 500 µl, der eine gerinnungshemmende Substanz zugegeben sein kann, von oben in den offenen Aufgaberaum 1, auf die Oberfläche der Membran 2 aufgegeben werden.

Das Vollblut gelangt vertikal durch die hier horizontal ausgerichtete Membran 2, wobei sich die hydrostatischen Kräfte zur Beschleunigung der Separation des Blutplasma, die unter Nutzung chromatografischer Effekte der Membran 2 erfolgt, zeitverkürzend auswirken. Das gegenüber den Erythrozythen und anderen im Vollblut enthaltenden zellulären Bestandteilen schnell durch die Membran hindurchgehende Blutplasma wird aus der Unterseite der Membran 2 von der Transportmembran 5, deren Kapillarkräfte größer sind, aufgenommen und mit Hilfe von Kapillarkräften strömt es lateral in Richtung des sich verjüngenden Bereiches und demzufolge zur Öffnung des Flußkanals 4. Dort kann es mit der erwähnten Spritze, unter Ausnutzung einer Saugkraft entnommen werden.

Mit der beschriebenen Anordnung können aus der 500 µl Vollblutprobe ca. 50 µl Blutplasma in ca. 5 min erhalten werden.

Mit der Abdeckung 11 kann der Aufgaberaum 1 abgedeckt und über die in der Abdeckung 11 ausgebildete Öffnung 12 die Flüssigkeit in den Aufgaberaum 1 aufgegeben werden. Dadurch kann ein Verschütten von Probenflüssigkeit vermieden werden.

Mit einer solchen Ausbildung kann über die Öffnung 12 auch eine Druckkraft ausgeübt werden. Dazu kann z.B. in die Öffnung 12 eine mit Luft aufgezogene Spritze, als ein Beispiel für eine Kolben-Zylindereinheit, eingeführt oder dort angesetzt werden. Beim Bewegen des Spritzenkolbens wird Luft oberhalb der Probenflüssigkeit in den Aufgaberaum 1 gepreßt und eine Druckkraft ausgeübt.

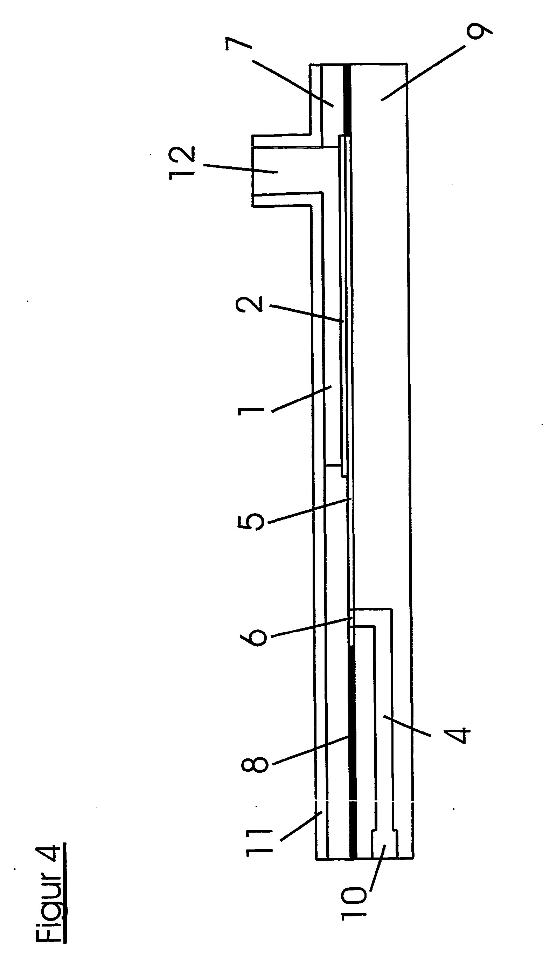

Mit der Schnittdarstellung gemäß Figur 4 soll insbesondere die Anordnung einer Transportmembran 5 im Hohlraum 3 geringer Höhe verdeutlicht werden, wobei bei der hier verwendeten Transportmembran 5 zusätzlich zu der ihr innewohnenden Kapillarkraftwirkung auch eine zusätzliche Separationsfunktion für ungelöste Bestandteile erreicht wird.

Zur Separationsunterstützung kann durch Ansatz einer Kolben-Zylindereinheit an zumindest eine der Öffnungen 10 oder 12 entweder eine Saugkraft an der Öffnung 10 oder eine Druckkraft an der Öffnung 12 erzeugt werden. Dabei kann mit einer solchen Kolben-Zylindereinheit eine Relativbewegung zwischen Kolben - und Zylinder in kontinuierlicher Form, in einer schrittweisen Bewegung in zumindest zwei Stufen oder eine von einem Endanschlag begrenzte Bewegung durchgeführt und demzufolge die Saug- oder Druckkraft entsprechend erzeugt werden.

Mit einer solchen Vorrichtung kann mit Saug- und/oder Druckkraftunterstützung die Separation von Blutplasma aus Vollblut in einem Zeitraum von maximal 10 min durchgeführt werden, wobei beispielsweise bei einer Vollblutmenge von 550 µl, das mit Saarstedt-Monovetten heparinisiert worden ist, eine Plasmaausbeute von bis zu 20 % erreicht werden kann.

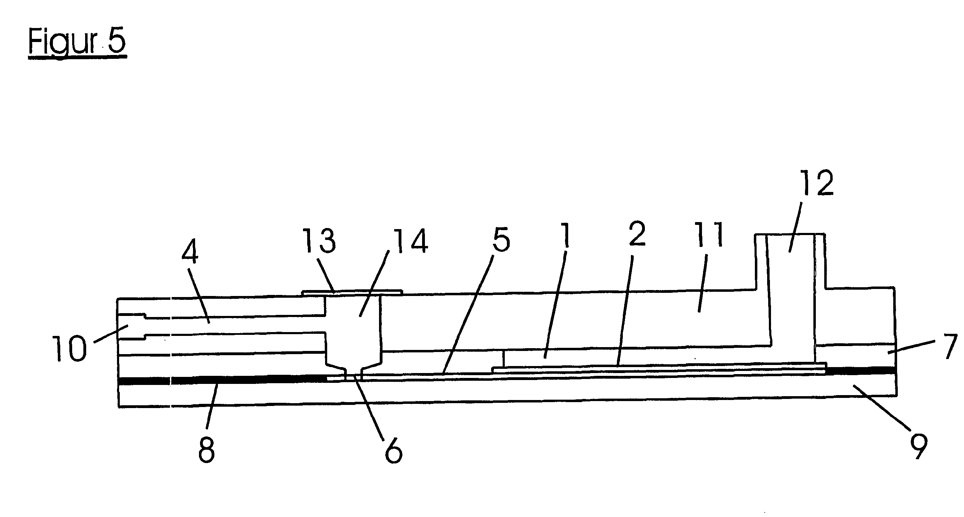

Bei dem in einer Schnittdarstellung in Figur 5 gezeigten Beispiel einer erfindungsgemäßen Vorrichtung ist ein zusätzlicher Zwischenbehälter 14 für separierte Flüssigkeit an den Hohlraum 3 geringer Höhe angeschlossen. Die Eintrittsöffnung für die von ungelösten Bestandteilen befreite biologische Flüssigkeit in den Zwischenbehälter 14 ist an der in der Transportmembran 5 ausgebildeten Öffnung 6 angeordnet.

Der Zwischenbehälter 14 verfügt über eine Öffnung, durch die die separierte Flüssigkeit, beispielsweise für die Durchführung nachfolgender Analysen, aus der die von ungelösten Bestandteilen befreite Flüssigkeit mit einer Pipette oder einer herkömmlichen Spritze mit Kanüle entnommen werden kann.

Der Zwischenbehälter 14 sollte mit einer zumindest flüssigkeitsdichten Abdeckung 13 nach außen temporär verschlossen werden. Eine solche Abdeckung 13 kann beispielsweise eine Folie sein, die umlaufend in einem Randbereich mit einem Haftvermittler versehen und so auf die Abdeckung bzw. auch ein Deckelteil 7 zum temporären Verschließen der Öffnung des Zwischenbehälters 14 aufgeklebt werden kann.

Für den Fall, dass der Zwischenbehälter 14 keine weitere Verbindung zur Umgebung hat und die Separation mit einer Druckkraftunterstützung durchgeführt werden soll, ist es günstig, diese Abdeckung zwar flüssigkeitsdicht jedoch gaspermeabel auszubilden.

In der in Figur 5 gezeigten Form ist dies jedoch nicht unbedingt erforderlich, da der Zwischenbehälter 14 mit dem Flußkanal 4 verbunden ist und am Flußkanal 4 eine Öffnung 10 vorhanden ist. Bei einer solchen Ausbildung kann auch zusätzlich mit Saugkraftunterstützung separiert werden, wie dies bereits bei den anderen Beispielen und im allgemeinen Teil der Beschreibung erläutert worden ist.

Zur Vermeidung des Eintritts und Abfließens von bereits separierter Flüssigkeit aus dem Zwischenbehälter 14 durch den Flußkanal 4 und der Öffnung 10 kann die Eintrittsöffnung des Flußkanals 4 am Zwischenbehälter 14 so angeordnet werden, dass der Pegel der separierten Flüssigkeit die Eintrittsöffnung des Flußkanals 4 nicht erreicht. Eine andere Alternative zur Verhinderung dieses Effektes besteht in der Verwendung einer flüssigkeitsdichten und gaspermeablen Membran, die an der Eintrittsöffnung oder im Inneren des Flußkanals 4 angeordnet sein kann.

Neben der Verwendung einer Folie als Abdeckung 13 für die Öffnung des Zwischenbehälters 14 kann aber auch ein kraft- und/oder formschlüssig fixierbarer Deckel, beispielsweise aus einem Kunststoffmaterial, der einfach in die Öffnung eingepresst werden kann, verwendet werden.

Ein solcher Deckel kann für die Entnahme von separierter Flüssigkeit aus dem Zwischenbehälter 14 relativ einfach wieder entfernt werden, oder es besteht außerdem die Möglichkeit, diesen Deckel, als Abdekkung 13 mit einer Kanüle einer herkömmlichen Spritze zu durchstechen und so die separierte Flüssigkeit aus dem Zwischenbehälter 14 abzuziehen, was sinngemäß auch auf die Verwendung einer Folie, als Abdeckung 13 zutrifft.

## Patentansprüche

1. Vorrichtung zur Separation von ungelösten Bestandteilen aus biologischen Flüssigkeiten, wobei zwischen einem Aufgaberaum (1) für die Flüssigkeit und einem Flusskanal (4) oder einer Öffnung ein Hohlraum (3) geringer Höhe, die kleiner als 1 mm ist, angeordnet und eine in lateraler Richtung die ungelösten Bestandteile separierende und zum Flusskanal (4) oder Öffnung transportierende Transportmembran (5) zumindest im Hohlraum (3) angeordnet ist.

2. Vorrichtung nach Anspruch 1,
**dadurch gekennzeichnet, dass** der Aufgaberaum (1) für die Flüssigkeit und der Hohlraum (3) geringer Höhe mittels einer flächigen, die ungelösten Bestandteile separierenden Membran (2), durch die die biologische Flüssigkeit in orthogonaler Richtung in den Hohlraum (3) geringer Höhe gelangt, getrennt sind.

3. Vorrichtung nach Anspruch 1 oder 2,
**dadurch gekennzeichnet, dass** der Hohlraum (3) geringer Höhe sich in Richtung auf den angeschlossenen Flusskanal (4) oder die Öffnung verjüngend ausgebildet ist.

4. Vorrichtung nach Anspruch 3,
**dadurch gekennzeichnet, dass** der sich verjüngende Bereich des Hohlraums (3) geringer Höhe, der an den Flusskanal (4) oder die Öffnung angeschlossen ist, außerhalb des von der separierenden Membran (2) abgedeckten Bereiches angeordnet ist.

5. Vorrichtung nach einem der Ansprüche 2 bis 4,
**dadurch gekennzeichnet, dass** die separierende Membran (2) aufgrund chromatographischer Effekte separiert.

6. Vorrichtung nach einem der Ansprüche 2 bis 5,
**dadurch gekennzeichnet, dass** die im Hohlraum (3) geringer Höhe angeordnete Transportmembran (5) mit höherer Kapillarkraftwirkung, als die der ausschließlich separierenden Membran (2), flächig mit der separierenden Membran (2) kontaktiert ist.

7. Vorrichtung nach einem der Ansprüche 2 bis 5,
**dadurch gekennzeichnet, dass** die den Aufgaberaum (1) und den Hohlraum (3) geringer Höhe trennende, separierende Membran (2) eine mehrschichtige Polymermembran ist.

8. Vorrichtung nach einem der Ansprüche 1 bis 7,
**dadurch gekennzeichnet, dass** die separierende Transportmembran (5) den Hohlraum (3) und die gesamte Fläche des Aufgaberaumes(1) flächig ausfüllt.

9. Vorrichtung nach einem der Ansprüche 1 bis 8,
**dadurch gekennzeichnet, dass** die im Hohlraum (3) geringer Höhe angeordnete Transportmembran (5) den Hohlraum (3) passgenau ausfüllt und der Form des Hohlraumes (3) angepasst ist.

10. Vorrichtung nach einem der Ansprüche 1 bis 9,
**dadurch gekennzeichnet, dass** in der Transportmembran (5) eine Öffnung (6) um den Flusskanal (4) oder der weiteren Öffnung ausgebildet ist.

11. Vorrichtung nach einem der Ansprüche 1 bis 10,
**dadurch gekennzeichnet, dass** am Ausgang des Hohlraums (3) geringer Höhe ein eine Saugkraft erzeugendes Element anschließbar ist.

12. Vorrichtung nach einem der Ansprüche 1 bis 11,
**dadurch gekennzeichnet, dass** das die Saugkraft erzeugende Element an den Flusskanal (4) oder eine am Hohlraum (3) geringer Höhe angeordnete Öffnung anschließbar ist.

13. Vorrichtung nach Anspruch 11 oder 12,
**dadurch gekennzeichnet, dass** das die Saugkraft erzeugende Element, eine Kolben-Zylindereinheit darstellt und der Zylinder die separierte biologische Flüssigkeit aufnimmt.

14. Vorrichtung nach einem der Ansprüche 1 bis 13,
**dadurch gekennzeichnet, dass** am Aufgaberaum (1) ein eine Druckkraft erzeugendes Element angeordnet oder dort anschließbar ist.

15. Vorrichtung nach einem der Ansprüche 1 bis 14,
**dadurch gekennzeichnet, dass** der Aufgaberaum (1) mit einem Deckel (11), in dem eine Öffnung (12) ausgebildet ist, abgeschlossen ist.

16. Vorrichtung nach Anspruch 14 oder 15,
**dadurch gekennzeichnet, dass** an die Öffnung (12) ein eine Druckkraft erzeugendes Element anschließbar ist.

17. Vorrichtung nach Anspruch 13 his 16,
**dadurch gekennzeichnet, dass** das eine Druckkraft erzeugende Element eine Kolben-Zylindereinheit ist.

18. Vorrichtung nach einem der Ansprüche 1 bis 17,
**dadurch gekennzeichnet, dass** der Hohlraum (3) geringer Höhe eine Höhe im Bereich zwischen 0,01 und 0,5 mm aufweist.

19. Vorrichtung nach einem der Ansprüche 1 bis 18,
**dadurch gekennzeichnet, dass** im Hohlraum (3) geringer Höhe kapillarförmige Kanäle ausgebildet sind, die in den Flusskanal (4) oder eine Öffnung münden.

20. Vorrichtung nach einem der Ansprüche 1 bis 19,
**dadurch gekennzeichnet, dass** der Aufgaberaum (1) in einem Deckelteil (7) ausgebildet ist, das mit einem Klebefilm (8), in dem der Hohlraum (3) ausgebildet ist, mit einem Basisteil (9), in dem ein Flusskanal (4) oder eine Öffnung ausgebildet ist, verbunden ist.

21. Vorrichtung nach einem der Ansprüche 1 bis 20,
**dadurch gekennzeichnet, dass** der Hohlraum (3) außer im Bereich des Aufgaberaumes (1) und einer Öffnung oder im Bereich der Öffnung (6) der Transportmembran (5) flüssigkeitsdicht abgeschlossen ist.

22. Vorrichtung nach einem der Ansprüche 1 bis 21,
**dadurch gekennzeichnet, dass** ein Zwischenbehälter (14) für separierte Flüssigkeit an den Hohlraum (3) geringer Höhe angeschlossen ist.

23. Vorrichtung nach Anspruch 22,
**dadurch gekennzeichnet, dass** der zwischenbehälter (14) zwischen der Öffnung, dem Flusskanal (4) und dem Hohlraum (3) geringer Höhe angeordnet ist.

24. Vorrichtung nach Anspruch 22 oder 23, **dadurch gekennzeichnet, dass** eine Öffnung zur Entnahme separierter Flüssigkeit des Zwischenbehälters (14) mit einer Abdeckung (13) verschlossen ist.

25. Vorrichtung nach Anspruch 24,
**dadurch gekennzeichnet, dass** die Abdeckung (14) aus einem flüssigkeitsdichten Material besteht.

26. Vorrichtung nach Anspruch 24 oder 25, **dadurch gekennzeichnet, dass** die Abdeckung (14) gaspermeabel ist.

27. Verfahren zur Separation von ungelösten Bestandteilen aus biologischen Flüssigkeiten, wobei die biologische Flüssigkeit in einen Aufgaberaum (1) gegeben,
aus dem Aufgaberaum (1) in eine in lateraler Richtung ungelöste Bestandteile separierende Transportmembran (5) gelangt und dabei
durch Kapillarkräfte der Transportmembran (5) transversal durch einen Hohlraum (3) geringer Höhe, die kleiner als 1 mm ist, als reine Flüssigkeit in ein Volumen überführt wird.

28. Verfahren nach Anspruch 27,
**dadurch gekennzeichnet, dass** die in den Aufgaberaum (1) gegebene biologische Flüssigkeit
in orthogonaler Richtung durch eine die biologische Flüssigkeit von ungelösten Bestandteilen separierende Membran (2),
aus der Membran (2) über den Hohlraum (3) mit geringer Höhe, als reine Flüssigkeit in ein Volumen überführt wird.

29. Verfahren nach Anspruch 27 oder 28,
**dadurch gekennzeichnet, dass** die in den Aufgaberaum (1) gegebene biologische Flüssigkeit mittels Saug-, Druck-, Kapillarkräften und/oder dem hydrostatischen Druck der Flüssigkeitssäule, als reine Flüssigkeit in ein Volumen überführt wird.

30. Verfahren nach einem der Ansprüche 27 bis 29,
**dadurch gekennzeichnet, dass** der Transport und die Separation der biologischen Flüssigkeit durch auf die in den Aufgaberaum (1) gegebene biologische Flüssigkeit wirkende Druckkräfte unterstützt wird.

31. Verfahren nach Anspruch 30,
**dadurch gekennzeichnet, dass** die Druckkraft über eine Öffnung (12), die in einem den Aufgaberaum (1) abschließenden Deckel (11) ausgebildet ist, ausgeübt wird.

32. Verfahren nach einem der Ansprüche 27 bis 31,
**dadurch gekennzeichnet, dass** die Druckkräfte mit einer Kolben-Zylindereinheit erzeugt werden.

33. Verfahren nach einem der Ansprüche 28 bis 32,
**dadurch gekennzeichnet, dass** die biologische Flüssigkeit mittels eines Saugkräfte erzeugenden Elementes saugkraftunterstützt separiert, aus der Membran (2) in den Hohlraum (3) oder die Transportmembran (5) gelangt, zu einem Flusskanal (4) oder einer Öffnung transportiert und die separierte biologische Flüssigkeit in ein Volumen aufgenommen wird.

34. Verfahren nach Anspruch 33,
**dadurch gekennzeichnet, dass** die Saugkräfte mit einer Kolben-Zylindereinheit erzeugt werden und die separierte biologische Flüssigkeit im Zylinder der Kolben-Zylindereinheit aufgenommen wird.

## Claims

1. Device for separating undissolved constituents out of biological fluids, in which device a cavity (3) of small height, less than 1 mm, is arranged between a feed chamber (1) for the fluid and a flow channel (4) or an opening, and a transport membrane (5) separating the undissolved constituents in the lateral direction and transporting them to the flow channel (4) or opening is arranged at least in the cavity (3).

2. Device according to Claim 1, **characterized in that** the feed chamber (1) for the fluid and the cavity (3) of small height are divided from one another by means of a planar membrane (2) which separates the undissolved constituents and through which the biological fluid passes in an orthogonal direction into the cavity (3) of small height.

3. Device according to Claim 1 or 2, **characterized in that** the cavity (3) of small height is designed narrowing in the direction towards the connected flow channel (4) or the opening.

4. Device according to Claim 3, **characterized in that** the narrowing area of the cavity (3) of small height which is connected to the flow channel (4) or to the opening is arranged outside the area covered by the separating membrane (2).

5. Device according to one of Claims 2 to 4, **characterized in that** the separating membrane (2) separates on the basis of chromatographic effects.

6. Device according to one of Claims 2 to 5, **characterized in that** the transport membrane (5) arranged in the cavity (3) of small height, and having a greater capillary force action than that of the exclusively separating membrane (2), is in planar contact with the separating membrane (2).

7. Device according to one of Claims 2 to 5, **characterized in that** the separating membrane (2) dividing the feed chamber (1) from the cavity (3) of small height is a multi-layer polymer membrane.

8. Device according to one of Claims 1 to 7, **characterized in that** the separating transport membrane (5) covers the cavity (3) and the whole surface of the feed chamber (1).

9. Device according to one of Claims 1 to 8, **characterized in that** the transport membrane (5) arranged in the cavity (3) of small height fills the cavity (3) with an exact fit and is adapted to the shape of the cavity (3).

10. Device according to one of Claims 1 to 9, **characterized in that**, in the transport membrane (5), an opening (6) is formed around the flow channel (4) or the further opening.

11. Device according to one of Claims 1 to 10, **characterized in that** an element generating a suction force can be connected at the outlet of the cavity (3) of small height.

12. Device according to one of Claims 1 to 11, **characterized in that** the element generating the suction force can be connected to the flow channel (4) or to an opening arranged on the cavity (3) of small height.

13. Device according to Claim 11 or 12, **characterized in that** the element generating the suction force is a piston/cylinder unit, and the cylinder receives the separated biological fluid.

14. Device according to one of Claims 1 to 13, **characterized in that** an element generating a pressure force is arranged on the feed chamber (1) or can be connected there.

15. Device according to one of Claims 1 to 14, **characterized in that** the feed chamber (1) is closed with a lid (11) in which an opening (12) is formed.

16. Device according to Claim 14 or 15, **characterized in that** an element generating a pressure force can be connected to the opening (12).

17. Device according to Claims 13 to 16, **characterized in that** the element generating a pressure force is a piston/cylinder unit.

18. Device according to one of Claims 1 to 17, **characterized in that** the cavity (3) of small height is between 0.01 and 0.5 mm high.

19. Device according to one of Claims 1 to 18, **characterized in that** capillary channels are formed in the cavity (3) of small height and open into the flow channel (4) or into an opening.

20. Device according to one of Claims 1 to 19, **characterized in that** the feed chamber (1) is formed in a lid part (7) which is connected by an adhesive film (8), in which the cavity (3) is formed, to a base part (9) in which a flow channel (4) or an opening is formed.

21. Device according to one of Claims 1 to 20, **characterized in that** the cavity (3) is sealed off in a fluid-tight manner except in the area of the feed chamber (1) and of an opening or in the area of the opening (6) of the transport membrane (5).

22. Device according to one of Claims 1 to 21, **characterized in that** an intermediate container (14) for separated fluid is connected to the cavity (3) of small height.

23. Device according to Claim 22, **characterized in that** the intermediate container (14) is arranged between the opening, the flow channel (4) and the cavity (3) of small height.

24. Device according to Claim 22 or 23, **characterized in that** an opening for removal of separated fluid from the intermediate container (14) is closed by a cover (13).

25. Device according to Claim 24, **characterized in that** the cover (13) is made of a fluid-tight material.

26. Device according to Claim 24 or 25, **characterized in that** the cover (13) is gas-permeable.

27. Method for separating undissolved constituents out of biological fluids, in which method the biological fluid is introduced into a feed chamber (1),
passes from the feed chamber (1) into a transport membrane (5) separating undissolved constituents in the lateral direction, and
by means of capillary forces of the transport membrane (5), is transferred transversely through a cavity (3) of small height, less than 1 mm, as pure fluid into a receiving volume.

28. Method according to Claim 27, **characterized in that** the biological fluid introduced into the feed chamber (1),
passes in an orthogonal direction through a membrane (2) separating the biological fluid from undissolved constituents;
and is transferred from the membrane (2), through the cavity (3) of small height, as a pure fluid into a receiving volume.

29. Method according to Claim 27 or 28, **characterized in that** the biological fluid introduced into the feed chamber (1) is transferred as pure fluid into a receiving volume by means of suction forces, pressure forces, capillary forces and/or the hydrostatic pressure of the liquid column.

30. Method according to one of Claims 27 to 29, **characterized in that** the transport and separation of the biological fluid is assisted by pressure forces acting on the biological fluid introduced into the feed chamber (1).

31. Method according to Claim 30, **characterized in that** the pressure force is exerted via an opening (12) which is formed in a lid (11) closing the feed chamber (1).

32. Method according to one of Claims 27 to 31, **characterized in that** the pressure forces are generated with a piston/cylinder unit.

33. Method according to one of Claims 28 to 32, **characterized in that** the biological fluid is separated with suction force assistance by means of an element generating suction forces, is passed from the membrane (2) into the cavity (3) or the transport membrane (5), and is transported to a flow channel (4) or to an opening, and the separated biological fluid is received in a receiving volume.

34. Method according to Claim 33, **characterized in that** the suction forces are generated with a piston/cylinder unit, and the separated biological fluid is received in the cylinder of the piston/cylinder unit.

## Revendications

1. Dispositif pour séparer des constituants non dissous contenus dans des liquides biologiques, dans lequel est ménagée, entre une zone d'alimentation (1) pour le liquide et un canal d'écoulement (4) ou une ouverture, une cavité (3) de faible hauteur, qui est inférieure à 1 mm, et où une membrane de transport (5) séparant les constituants non dissous dans la direction latérale et les transportant vers le canal d'écoulement (4) ou l'ouverture est disposée au moins dans la cavité (3).

2. Dispositif selon la revendication 1, **caractérisé en ce que** la zone d'alimentation (1) destinée au liquide et la cavité (3) de faible hauteur sont séparées au moyen d'une membrane plane (2) séparant les constituants non dissous, à travers laquelle membrane le liquide biologique atteint, en direction orthogonale, la cavité (3) de faible hauteur.

3. Dispositif selon l'une quelconque des revendications 1 ou 2, **caractérisé en ce que** la cavité (3) de faible hauteur présente une forme qui s'amincit en direction du canal d'écoulement raccordé (4) ou de l'ouverture.

4. Dispositif selon la revendication 3, **caractérisé en ce que** la zone amincie de la cavité (3) de faible hauteur, qui est raccordée au canal d'écoulement (4) ou à l'ouverture, est aménagée en dehors de la zone recouverte par la membrane séparatrice (2).

5. Dispositif selon l'une quelconque des revendications 2 à 4, **caractérisé en ce que** la membrane séparatrice (2) effectue une séparation en raison d'effets chromatographiques.

6. Dispositif selon l'une quelconque des revendications 2 à 5, **caractérisé en ce que** la membrane de transport (5), aménagée dans la cavité (3) de faible hauteur et ayant un effet de capillarité plus élevé que celui de la membrane exclusivement séparatrice (2), est mise au contact à plat avec la membrane séparatrice (2).

7. Dispositif selon l'une quelconque des revendications 2 à 5, **caractérisé en ce que** la membrane séparatrice (2), séparant la zone d'alimentation (1) et la cavité (3) de faible hauteur, est une membrane polymère multicouche.

8. Dispositif selon l'une quelconque des revendications 1 à 7, **caractérisé en ce que** la membrane de transport séparatrice (5) remplit à plat la cavité (3) et toute la surface de la zone d'alimentation.

9. Dispositif selon l'une quelconque des revendications 1 à 8, **caractérisé en ce que** la membrane de transport (5), aménagée dans la cavité (3) de faible hauteur, remplit avec précision la cavité (3) et est adaptée à la forme de la cavité (3).

10. Dispositif selon l'une quelconque des revendications 1 à 9, **caractérisé en ce qu'**une ouverture (6) est ménagée dans la membrane de transport (5) autour du canal d'écoulement (4) ou de l'autre ouverture.

11. Dispositif selon l'une quelconque des revendications 1 à 10, **caractérisé en ce que**, à la sortie de la cavité (3) de faible hauteur, on peut raccorder un élément produisant une force d'aspiration.

12. Dispositif selon l'une quelconque des revendications 1 à 11, **caractérisé en ce que** l'élément produisant la force d'aspiration peut être raccordé au canal d'écoulement (4) ou à une ouverture ménagée sur la cavité (3) de faible hauteur.

13. Dispositif selon la revendication 11 ou 12, **caractérisé en ce que** l'élément produisant la force d'aspiration présente une unité à piston-cylindre et **en ce que** le cylindre recueille le liquide biologique séparé.

14. Dispositif selon l'une quelconque des revendications 1 à 13, **caractérisé en ce qu'**un élément produisant une force de pression est aménagé dans la zone d'alimentation (1) ou peut y être raccordé.

15. Dispositif selon l'une quelconque des revendications 1 à 14, **caractérisé en ce que** la zone d'alimentation (1) est fermée par un couvercle (11), dans lequel est ménagée une ouverture (12).

16. Dispositif selon la revendication 14 ou 15, **caractérisé en ce que** l'on peut raccorder un élément produisant une force de pression à l'ouverture (12).

17. Dispositif selon l'une quelconque des revendications 13 à 16, **caractérisé en ce que** l'élément produisant une force de pression est une unité à piston-cylindre.

18. Dispositif selon l'une quelconque des revendications 1 à 17, **caractérisé en ce que** la cavité (3) de faible hauteur présente une hauteur dans la plage comprise entre 0,01 et 0,5 mm.

19. Dispositif selon l'une quelconque des revendications 1 à 18, **caractérisé en ce que** sont élaborés dans la cavité (3) de faible hauteur des canaux en forme de capillaires, qui aboutissent dans le canal d'écoulement (4) ou dans une ouverture.

20. Dispositif selon l'une quelconque des revendications 1 à 19, **caractérisé en ce que** la zone d'alimentation (1) est formée dans une partie de couvercle (7), qui est relié, avec un film adhésif (8), dans lequel la cavité (3) est formée, à une partie de base (9), dans laquelle est formé(e) un canal d'écoulement (4) ou une ouverture.

21. Dispositif selon l'une quelconque des revendications 1 à 20, **caractérisé en ce que** la cavité (3) est fermée de manière étanche aux liquides à l'extérieur dans la région de la zone d'alimentation (1) et d'une ouverture ou dans la zone de l'ouverture (6) de la membrane de transport (5).

22. Dispositif selon l'une quelconque des revendications 1 à 21, **caractérisé en ce qu'**un réceptacle intermédiaire (14) pour un liquide séparé est raccordé à la cavité (3) de faible hauteur.

23. Dispositif selon la revendication 22, **caractérisé en ce que** le réceptacle intermédiaire (14) est aménagé entre l'ouverture, le canal d'écoulement (4) et la cavité (3) de faible hauteur.

24. Dispositif selon la revendication 22 ou 23, **caractérisé en ce qu'**une ouverture destinée au prélèvement du liquide séparé du réceptacle intermédiaire (14) est fermée par un couvercle (13).

25. Dispositif selon la revendication 24, **caractérisé en ce que** le couvercle (13) est constitué d'un matériau étanche aux liquides.

26. Dispositif selon la revendication 24 ou 25, **caractérisé en ce que** le couvercle (13) est perméable aux gaz.

27. Procédé pour séparer des constituants non dissous de liquides biologiques, dans lequel le liquide biologique est délivré dans une zone d'alimentation (1), parvient de la zone d'alimentation (1) dans une membrane de transport (5) séparant les constituants non dissous dans la direction latérale et est transféré par les effets capillaires de la membrane de transport (5) transversalement à travers une cavité (3) de faible hauteur, qui est inférieure à 1 mm, sous la forme d'un volume de liquide pur.

28. Procédé selon la revendication 27, **caractérisé en ce que** le liquide biologique délivré dans la zone d'alimentation (1) est transféré, dans la direction orthogonale, à travers une membrane (2) séparant le liquide biologique des constituants non dissous, et de la membrane (2) via la cavité (3) de faible hauteur sous la forme d'un volume de liquide pur.

29. Procédé selon la revendication 27 ou 28, **caractérisé en ce que** le liquide biologique délivré dans la zone d'alimentation (1) est transféré au moyen de forces d'aspiration, de pression, de capillarité et/ou au moyen de la pression hydrostatique de la colonne de liquide, dans un volume sous la forme d'un liquide pur.

30. Procédé selon l'une quelconque des revendications 27 à 29, **caractérisé en ce que** le transport et la séparation du liquide biologique sont soutenus par des forces de pression agissant sur le liquide biologique délivré dans la zone d'alimentation (1).

31. Procédé selon la revendication 30, **caractérisé en ce que** la force de pression est exercée sur une ouverture (12), qui est formée dans un couvercle (11) fermant la zone d'alimentation (1).

32. Procédé selon l'une quelconque des revendications 27 à 31, **caractérisé en ce que** les forces de pression sont produites par une unité à piston-cylindre.

33. Procédé selon l'une quelconque des revendications 28 à 32, **caractérisé en ce que** le liquide biologique est séparé par assistance de forces d'aspiration au moyen d'un élément produisant des forces d'aspiration, parvient de la membrane (2) dans la cavité (3) ou dans la membrane de transport (5), est transporté à un canal d'écoulement (4) ou à une ouverture et le liquide biologique séparé est recueilli sous la forme d'un volume.

34. Procédé selon la revendication 33, **caractérisé en ce que** les forces d'aspiration sont produites avec une unité à piston-cylindre et **en ce que** le liquide biologique séparé est recueilli dans le cylindre de l'unité à piston-cylindre.
